(19) Europäisches Patentamt / European Patent Office / Office européen des brevets

(11) **EP 3 993 257 A1**

(12) # EUROPEAN PATENT APPLICATION

(43) Date of publication:
**04.05.2022 Bulletin 2022/18**

(21) Application number: **20205379.9**

(22) Date of filing: **03.11.2020**

(51) International Patent Classification (IPC):
**H02P 29/024** (2016.01)   **H02P 6/08** (2016.01)
**H02P 6/182** (2016.01)   **H02P 8/38** (2006.01)
**A61M 5/142** (2006.01)

(52) Cooperative Patent Classification (CPC):
**H02P 29/024; A61M 5/14248; A61M 5/16831;
H02P 6/085; H02P 6/182; H02P 8/38**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Ypsomed AG
3401 Burgdorf (CH)**

(72) Inventors:
• **Kappeler, Krista
  3004 Bern (CH)**
• **Rytz, Bernhard
  3436 Zollbrück (CH)**

(74) Representative: **Meier Obertüfer, Jürg et al
Ypsomed AG
Brunnmattstrasse 6
3401 Burgdorf (CH)**

(54)    **SYSTEM AND METHOD TO DETECT DRIVE BLOCKAGE IN A DRUG DELIVERY DEVICE**

(57)    The present invention describes a system and a method for detecting blockage of an actuating assembly in a drug delivery device with a brushless DC or stepper motor. The Back EMF voltage is periodically measured at the driving contacts of the motor. A blockage indication value is periodically calculated based on the divergence of voltages measured at different driving contacts, and an alarming and/or mitigation action is initiated if the blockage indication value meets a predefined criterion such as exceeding a minimum or maximum value.

Fig. 10a

EP 3 993 257 A1

**Description**

FIELD OF THE INVENTION

The present invention relates to a drug delivery device with a brushless electrical motor and an actuating assembly, to a method to detect blockage of the actuating assembly, and to a system to provide blockage detection in such a drug delivery device.

BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Drug delivery devices include injection devices that are removed from the site of application after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens or infusion pumps. Alternatively, patch injectors, wearable injectors or wearable pumps are patched or adhered to the skin of the patient.

Common to all devices for subcutaneous drug delivery is a reservoir to store the fluid medicament, and a fluid path to bring the drug out of the device and into the subcutaneous tissue of a patient. In a majority of injecting or infusion devices the reservoir has a plunger which is mechanically advanced by an actuation assembly - in this case usually a plunger rod - to drive the fluid out of the reservoir into the fluid path and towards the patient. Alternatively, a pump mechanism such as a peristaltic, membrane or piston pump may be used to transport the fluid and effectuate the drug delivery, with a motor-driven actuation assembly generating the mechanical movement.

For drug delivery devices, blockage of the fluid delivery is a major problem which can occur anywhere in the fluid path, e.g. by cristallisation of particles in the drug. Such a blockage in the fluid path is also known as occlusion. For safe and reliable drug delivery, detection of blockages and occlusions are an essential requirement.

In many drug delivery devices, an electric motor is used as a driving means. This also means that some electronic circuitry is integrated to control the motion of the motor and consequently the drug delivery. Every blockage in the fluid path, within the reservoir or within the actuating assembly results in a change of torque or driving force at the output of the motor. Therefore, to minimise complexity and cost of such a system, control and supervision of the drug delivery is often realised directly at the motor, for example by analysing the supply current drawn by motor coils, to avoid extra cost for additional sensors such as optical sensors to supervise the motion of the rotor or pressure sensors to detect excess force in a stall condition.

EP 0341364 B1 describes a drug delivery device with a plunger moving assembly supervision based on the motor current. The motor current is measured during operation of the pump and integrated over a certain time to eliminate spikes. A blockage is reported if the integrated motor current is higher than a pre-defined threshold.

An alternative concept for motor control is known as "back electro-motive force" (Back EMF or BEMF). Sensors are placed at the contacts of an electric motor to sense the voltage induced by motor coils passing the sensor if the motor is rotating. By monitoring these voltages, the motion of the motor can be detected and also used to detect blockage of the delivery. This technique is of special interest if the motor is a stepping motor or a brushless electric (BLDC) motor, where the same coils can be used for driving the motor and for sensing the back EMF.

US 2003/0117100 A1 describes a motor control system for a stepping motor using EMF supervision and blockage detection.

US 9,509,243 B2 describes a linear actuation system with back EMF detection of lost steps.

All efforts to supervise the drug delivery and detect blockage back at the driving motor highly depend on the mechanical properties of the actuating assembly or plunger moving assembly. In a system with a rigid and play-free plunger moving assembly any fault along the drive train and the fluid path can immediately be detected at the motor using one of the known techniques. However, with increasing elasticity of the actuating assembly, fault detection such as detection of blockage and occlusion becomes unreliable or even impossible within the accuracy requirements of a drug delivery device. More specifically, in case of an occlusion in the example of a system with a non-rigid plunger moving assembly, Back EMF will not immediately show zero current (complete blockage), because the driving motor will need some time to compress the elasticity of the plunger moving assembly and suppress the rotor movement sufficiently to detect the fault.

WO 2017/017557 A1 describes a method to compensate mechanical play using Back EMF voltage analysis in an actuation system with an elastic element.

To achieve the accuracy of movement and/or fault detection required for a drug delivery device, additional sensors and/or mechanisms in the fluid path may be needed, increasing complexity and cost of the drug delivery system.

At the same time it is advantageous for the mechanical design to allow a controlled amount of elasticity in the plunger moving assembly rather than eliminate the elasticity to avoid the problems described above. The classical linear plunger rod may be replaced by a different mechanism, such as a curved chain of short sticks, a train of balls in a bearing or even a spring, opening up a wide range of new possibilities for the design of drug delivery devices which may be more compact, more ergonomical in outer shape or more cost efficient in manufacturing or use.

There is clearly a need for a system and a method to detect blockage and occlusion in a drug delivery device with a potentially elastic plunger moving assembly, where the blockage is detected directly at the driving motor and no extra sensor is introduced in the reservoir or fluid path.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

DESCRIPTION OF THE INVENTION

It is an objective of the invention to provide an improved system and method to detect blockage in a medical fluid delivery device. The improvement explicitly includes providing reliable detection of a blockage in a drug delivery device with a non-rigid actuating assembly. A preferred application of the improved blockage detection may for example be in a compact, body-worn drug delivery device with a reservoir and a plunger, where relaxed requirements for rigidity of the plunger moving assembly are most advantageous.

This objective is achieved by a drug delivery device with an electric stepper motor or brushless DC motor (31) operatively connected to the actuating assembly and comprising at least one driving phase, preferably two driving phases, each driving phase having a coil (40), a positive driving contact (41), and a negative driving contact (42) to drive the motor. The drug delivery device further comprises a voltage measurement circuitry (55) to measure the voltage at or between the driving contacts (41). The drug delivery device further comprises an electronic control unit (20) with a control circuitry (50), an electrical power source, a microprocessor, storage and software configured to control the commutation of the motor (31), to supervise the actuation of the drug delivery device and to initiate an alarming and/or mitigation action if a blockage of the actuating assembly (30) has been detected. This detection is realised with a novel method of evaluating the Back EMF voltage of the motor which includes the following steps:

Step 1: commutating the motor (31) by applying a pattern of positive driving voltages (61) to the driving contacts (41), preferably repeating a commutation cycle with a succession of all positive driving contacts (41) and all negative driving contacts (42).

Step 2: pausing the driving of at least one driving phase periodically, preferably twice per commutation cycle, further preferably pausing between driving the positive and driving the negative driving contact (42) of the same driving phase, to open a recurring measuring window (63) during which the driving contacts (41) of the paused driving phase are controlled by the voltage measuring circuitry (55) to measure a Back EMF voltage at or between said driving contacts (41).

Step 3: measuring, preferably during each measuring window (63), the voltage at or between driving contacts (41) of a driving phase and establishing, preferably once for each measuring window, a convergence value (80) representing the Back EMF voltage during said measuring window (63).

Step 4: calculating, preferably once for every commutation cycle k, a blockage indication value BI based on the divergence of convergence values CV measured at different driving contacts. Any mathematical expression may be used to assess said divergence, for example a quantification of maxima and minima, as in the formula $BI(k) = \max(CP(k) - CN(k))$, where CP is a convergence value assigned to a positive driving contact (41), CN is a convergence value assigned to a negative driving contact (42). For the purpose of formulating the calculation of BI, an assigning step may further be included, which comprises assigning each convergence value (80) to a positive or to a negative driving contact, preferably to the positive or to the negative driving contact of the driving phase paused to perform the voltage measurement, further preferably assigning the convergence value to the driving contact to which the driving pattern has last been applied before starting the measurement window the convergence value is representing; storing the convergence values in sequences per driving contact, preferably with driving phases A and B the sequences $CP_A$, $CP_B$ for convergence values at the positive driving contacts $P_A$, $P_B$ and the sequences $CN_A$, $CN_B$ for convergence values at the negative driving contacts $N_A$, $N_B$.

Step5: deciding that a blockage of the actuation assembly is present if the blockage indication value BI meets a predefined criterion, preferably if the blockage indication value BI exceeds a blockage threshold $\pm$MaxConvDiff for a specified minimum number of commutation cycles k within a specified minimum blockage duration; and

Step 6: initiating an alarming and/or mitigation action if a blockage has been detected.

In the drug delivery device outlined above, two main aspects of the present invention are evident: a method using steps 1 to 7 to detect blockage of the actuation assembly, and a system built into the medical device to implement said method. More details about both aspects are specified in corresponding dependent claims.

BRIEF DESCRIPTION OF THE DRAWINGS

The subject matter of the invention will be explained in more detail in the following text with reference to preferred exemplary embodiments which are illustrated in the attached drawings, in which:

Fig.1      depicts a patch injector according to the present invention;
Fig.2      depicts the patch injector partially opened to show the electronic control unit;
Fig.3      depicts a selection of the interior of the patch injector with the motor and the actuation assembly;
Fig.4a     depicts an exploded physical view of a brushless stepper motor with two phases;
Fig.4b     depicts the physical arrangement of magnetic poles in the motor;
Fig.4c     depicts a schematic illustration of the magnetic structure of the motor;
Fig.5a     depicts the schematic diagram of a unipolar single phase motor drive circuitry;
Fig.5b     depicts the schematic diagram of a bipolar dual phase motor drive circuitry;
Fig.6a     depicts a driving voltage pattern used to commute the motor with a Back EMF measuring window;
Fig.6b     depicts a commutation cycle with measuring windows;
Fig.7      depicts a detailed view of the Back EMF voltage during a measuring window;
Fig.8      depicts a set of convergence data used to determine a convergence value;
Fig.9a     depicts a set of sequences of convergence values for two phases;
Fig.9b     depicts a set of sequences of blockage indication values calculated as differences of convergence values;
Fig.10a    depicts a set of sequences of smoothed convergence values, differences and mean values for phase A;
Fig.10b    depicts a set of sequences of smoothed convergence values, differences and mean values for phase B;
Fig. 11    illustrates the normalisation of convergence values;
Fig.12a    depicts a sequence of normalised smoothed convergence values, and their difference , for phase A;
Fig.12b    depicts a sequence of normalised smoothed convergence values, and their difference , for phase B;
Fig.13a    depicts a sequence of blockage indication values based on a maximum difference of smoothed convergence values in individual phases;
Fig.13b    depicts a sequence of blockage indication values based on a maximum difference of smoothed and normalised convergence values in individual phases;
Fig.14a    depicts a sequence of blockage indication values based on a maximum difference of smoothed convergence values in a combination of multiple phases;
Fig.14b    depicts a sequence of blockage indication values based on a maximum difference of normalised convergence values in a combination of multiple phases;
Fig.15     depicts a sequence of blockage indication values based on a maximum square error of convergence values;

The reference symbols used in the drawings, and their primary meanings, are listed in summary form in the list of designations. In principle, identical parts are provided with the same reference symbols in the figures.

**EP 3 993 257 A1**

DETAILED DESCRIPTION OF PREFERRED EMBODIMENTS

At the core of the present invention is a system and a method to detect blockage of an actuating assembly in a medical device with a brushless electric motor by means of a novel way of evaluating Back EMF voltages. While blockage detection is a very common requirement in medical devices, the invention can be used for any kind of medical device, with any kind of brushless electric motor and any kind of actuating assembly. In this document, the invention is described in a preferred embodiment of a drug delivery device, more specifically in the example of a wearable patch injector for subcutaneous application of a fluid drug. Any person skilled in the art will have no difficulty applying the same invention to the design of a patch pump, mobile pump, stationary pump or any other medical device with an actuating assembly suitable for supervision by analysis of Back EMF signals.

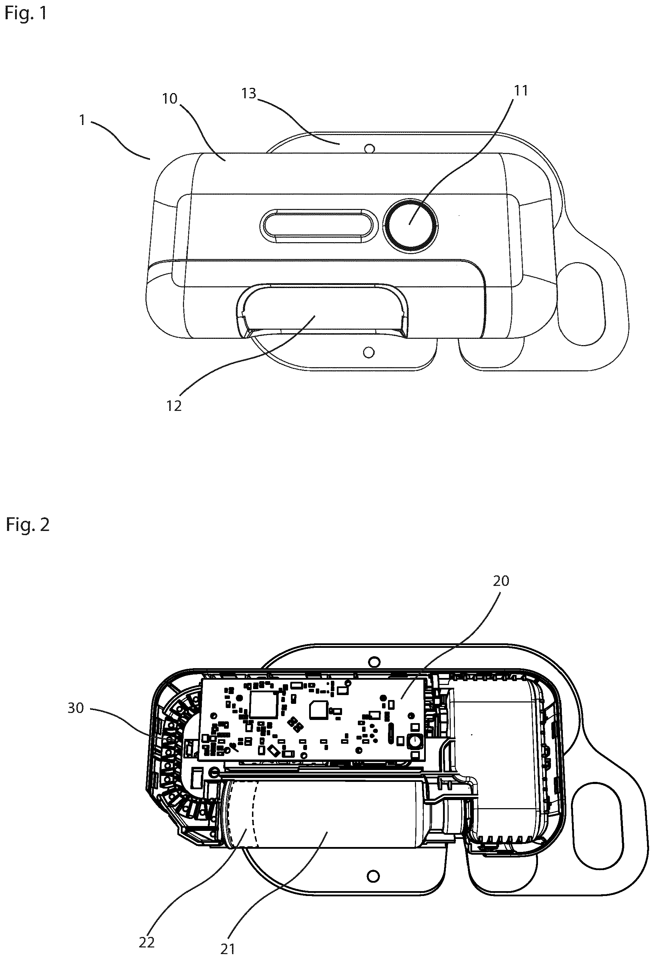

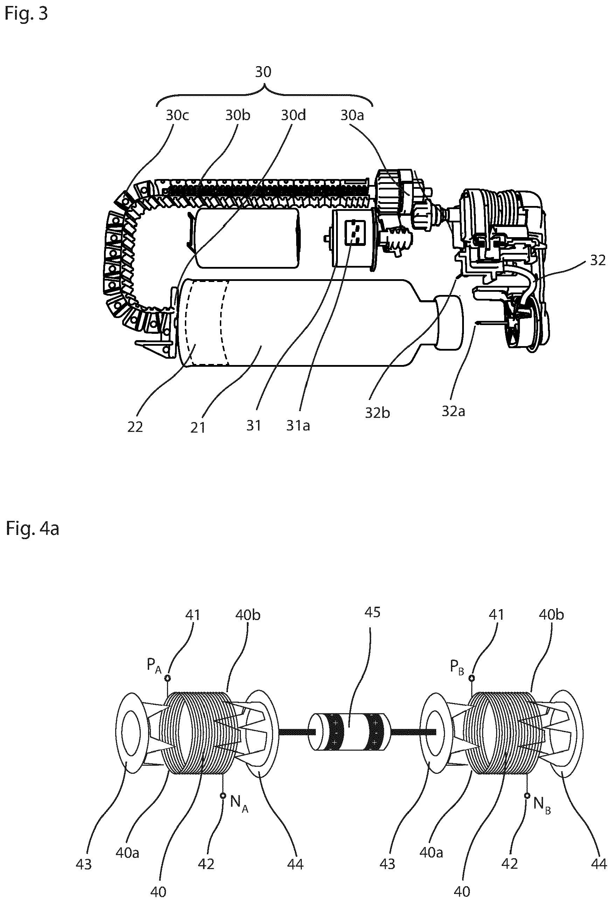

Figures 1 to 3 illustrate the patch injector (1) and the main components thereof. The patch injector (1) has a housing (10) to protect the device from the environment and provide a compact, wearable shape suitable for constant use over several minutes. An adhesive patch assembly (13) is permanently attached to the housing and used to attach the pump to the body of a patient for application. The housing holds a reservoir (21) to contain a medical fluid, a plunger (22) movably disposed in the reservoir (21), and a fluid transport assembly (32) to connect the reservoir to the patient. In this example, the reservoir has a septum and the fluid transport assembly (32) has a needle at the input (32a) to pierce said septum and connect the fluid transport assembly with the reservoir (21). At the output (32b) of the fluid transport assembly (32) is a cannula, configured to be temporarily inserted into the body of the patient for subcutaneous drug delivery. The drug delivery is effectuated by means of a brushless stepper motor (31) with a rotor (45) operatively connected to an actuating assembly (30, see Fig. 3 and 4a). The actuating assembly (30) comprises all components necessary to translate the movement of the rotor (45) to an advancement of the medical fluid. In this particular embodiment, the actuating assembly (30) is a plunger moving assembly comprising a gear box assembly (30a), a threaded rod (30b), a segmented rod (30c) and a plunger moving head (30d) mechanically connecting to the plunger (22) and moving the plunger (22) in the reservoir (21) forward. An electronic control unit with an electronic control circuitry (50), an electrical power source (not shown), hardware for electronic and/or visual and/or acoustic communication, a microprocessor, storage and software is packed into the housing and configured to control all system functions such as interacting with a user or interacting with another device involved in the drug delivery therapy, commutating the motor (31), supervising the actuation of the plunger and initiating an alarming action and/or a mitigation action if a blockage of the actuating assembly (30) has been detected. On the outside of the housing (10), a command button (11) is connected to the electronic control unit (20) to initiate drug delivery. A reservoir window (12) in the housing (10) may allow the patient to visually supervise the drug delivery by double-checking the position of the plunger (22) in the reservoir (21).

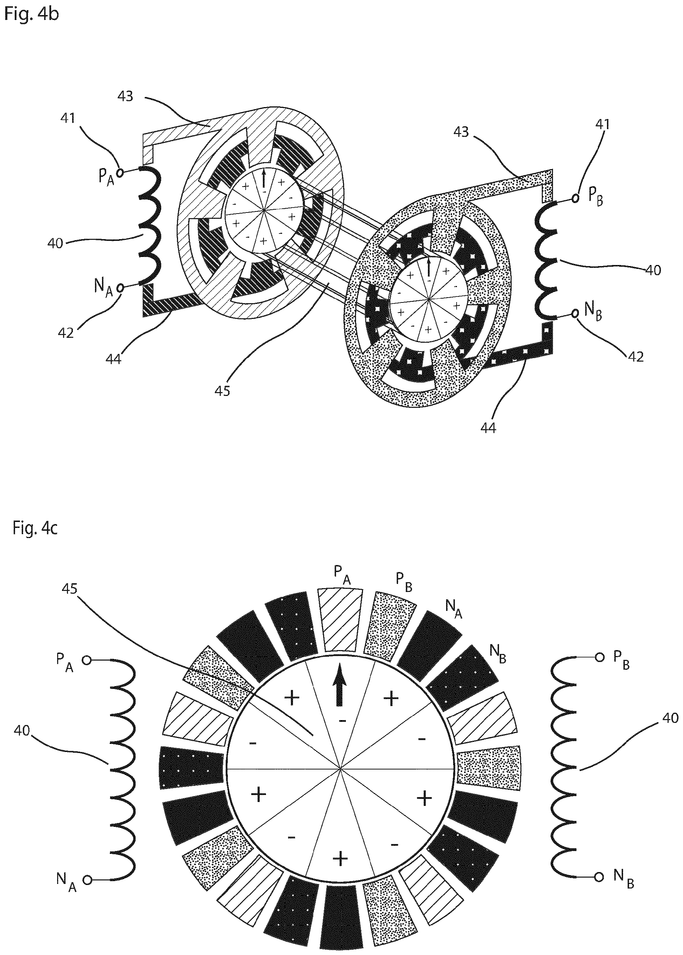

Figure 4a to 4c illustrate the brushless stepper motor used in a preferred embodiment of the invention. The motor has two driving phases A and B with two coils (40) radially arranged around the rotor (45), axially next to each other as shown in the exploded view of Fig. 4a. At both ends of each coil, a magnetic flux conductor (43, 44) collects the magnetic field induced by the coil when activated by applying an electric voltage to the driving contacts (41, 42) and distributes the magnetic flux to a number of protrusions, in the described embodiment five protrusions per magnetic flux conductor. The protrusions of the magnetic flux conductors (43, 44) are arranged inside the coil (40) alternately from both ends of the coil (40) to form a number of magnetic pole pairs (see Fig. 4b) used to drive the rotor (45), in the described embodiment five pole pairs per coil (45). The flux conductors of the two coils are arranged in a fixed position axially aligned around the axis of the rotor (45) and rotated by half the angle between two pole pairs, in the example given by 18 degrees. This arrangement forms a stator with two coils (40) and ten magnetic pole pairs as illustrated in the schematic view of Fig. 4c. For the purpose of describing the invention, we call one end of a coil (40) the positive end (40a) and the other end of the coil the negative end (40b). This definition is arbitrary, does not reflect any magnetic or electric polarity and could also be swapped between the two ends. Using this definition, each coil (40) has a positive driving contact P (41) and a positive magnetic flux conductor (44) at the positive end (40a), and a negative driving contact N (42) and a negative magnetic flux conductor (45) at the negative end (40b). In the example of the embodiment used to describe the invention, the driving phases A and B with coils A and B (40) have two positive driving contacts $P_A$, $P_B$ (41) and two negative driving contacts $N_A$, $N_B$ (42). By cyclically applying a positive voltage to a sequence of positive and negative driving contacts, the rotor is rotated or commutated continuously - in the case of a brushless DC motor - or in a number of discrete motor steps per rotation, in the example 20 motor steps per rotation. Alternative embodiments of the invention may have a motor with a different number of driving phases, such as single phase or triple phase, may have phases with a different number of coils, such as two coils in series or multiple coils in parallel, may have more than two driving contacts per driving phase, such as a middle contact $M_A$ between two coils connected in series.

The first three steps of blockage detection according to the present invention are tightly connected and will be described together in the following paragraphs. The steps are

1) to induce a movement of the rotor by applying a cyclic commutation at the driving contacts

2) periodically pause said commutation and open a measuring window

3) periodically sample the Back EMF voltage during said measuring window and obtain a digital convergence value

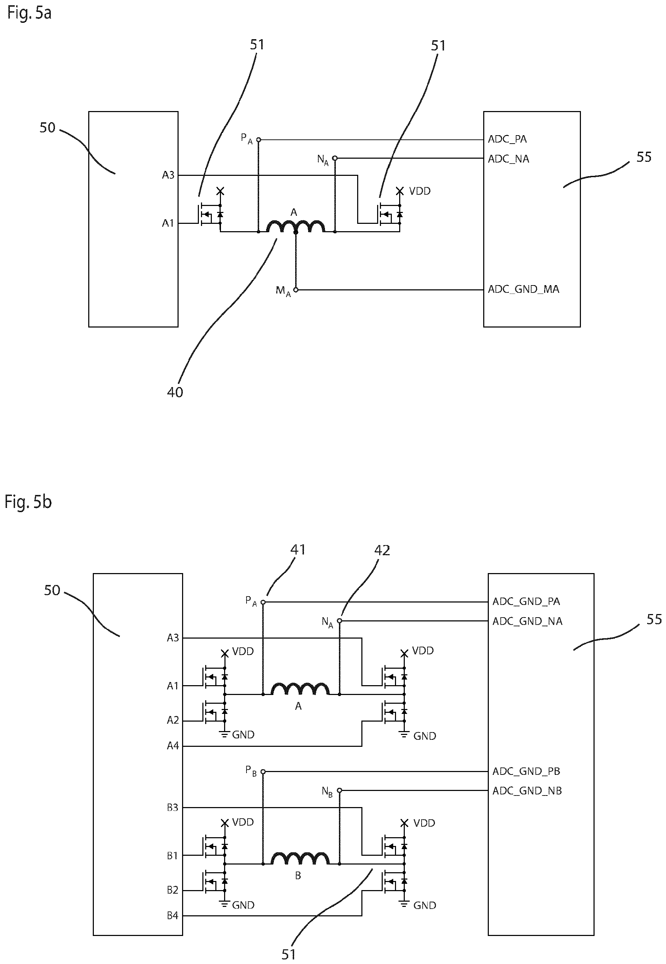

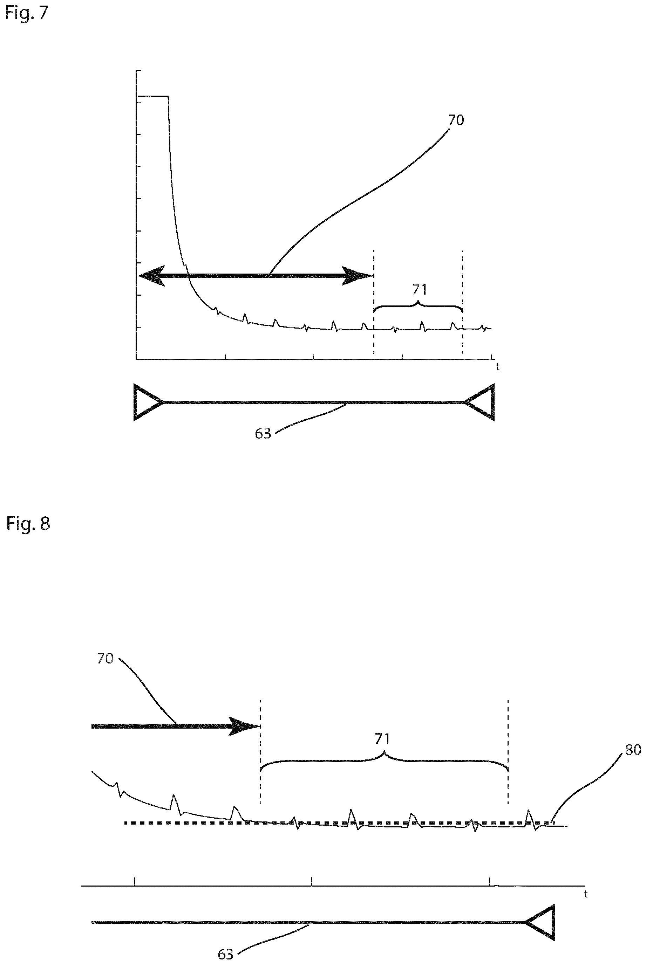

Electronic circuitry to commutate a brushless DC (BLDC) or stepper motor is well established in prior art. Fig. 5a shows a schematic diagram of a unipolar drive circuitry for a single phase motor, where the positive driving contact $P_A$ and the negative driving contact $N_A$ are connected to a positive driving voltage VDD via MOS-FET switches (51). The control circuitry (50) is configured to operate the switches and apply the driving voltage VDD alternately to $P_A$ and $N_A$ while the middle driving contact $M_A$ remains connected to the electrical ground GND. In the example of Fig. 5a, a voltage measuring circuitry is connected to all three driving contacts $P_A$, $N_A$ and $M_A$. This allows the control circuitry to pause the commutation and use the voltage measurement circuitry to sample the course of voltage at the driving contacts and determine the voltage induced by back electro-motive force (Back EMF). Voltage sampling is typically performed by repeatedly and synchronously triggering a number of analog-digital converters (ADC) in the measurement circuitry. The Back EMF voltages may simply be read from an ADC at a particular driving contact or calculated from a multitude of voltages sampled at a multitude of driving contacts of the same driving phase, for example by subtracting the voltage at $N_A$ from the voltage at $P_A$. The measurement circuitry may connect select driving contacts to VDD or to GND to control the sampling of Back EMF voltages. Instead of Back EMF voltages, Back EMF currents may be sampled in the same way. In both cases the sampled values are analysed, preferably once for each measuring window, and processed to obtain one digital convergence value per measuring window (80, Fig. 8), representative of the Back EMF signal during said measuring window.

In Fig. 5b, the driving and measuring circuitry is extended to an embodiment with a two-phase motor and a bipolar commutation. Each driving phase A, B has a coil (40) and four MOS-FET switches (51) forming a H-bridge. The control circuitry (50) is configured to operate the switches (51) and to apply a positive driving voltage cyclically to all positive and negative driving contacts, for example by repeating the sequence $P_A$, $P_B$, $N_A$, $N_B$. The sequence of driving contacts may vary with the design of the motor and with the direction of rotation. Whenever a driving contact is connected to VDD, the drive contact at the opposite end of the same coil is connected to the electrical ground GND. In this embodiment, as there are four driving contacts, the sequence includes four motor steps until the sequence is repeated, defining a commutation cycle of four motor steps. The direction of rotation can also be controlled by choosing the sequence of driving contacts in a commutation cycle according to the design of the motor. Just like with the unipolar operation, pauses in commutation allow the voltage measurement circuitry in Fig. 5b to connect the driving contacts (41, 42) to VDD or GND, to sample the voltages at the driving contacts, and to obtain one digital convergence value (80, Fig. 8) per measuring window (63), representative of the Back EMF signal during said measuring window.

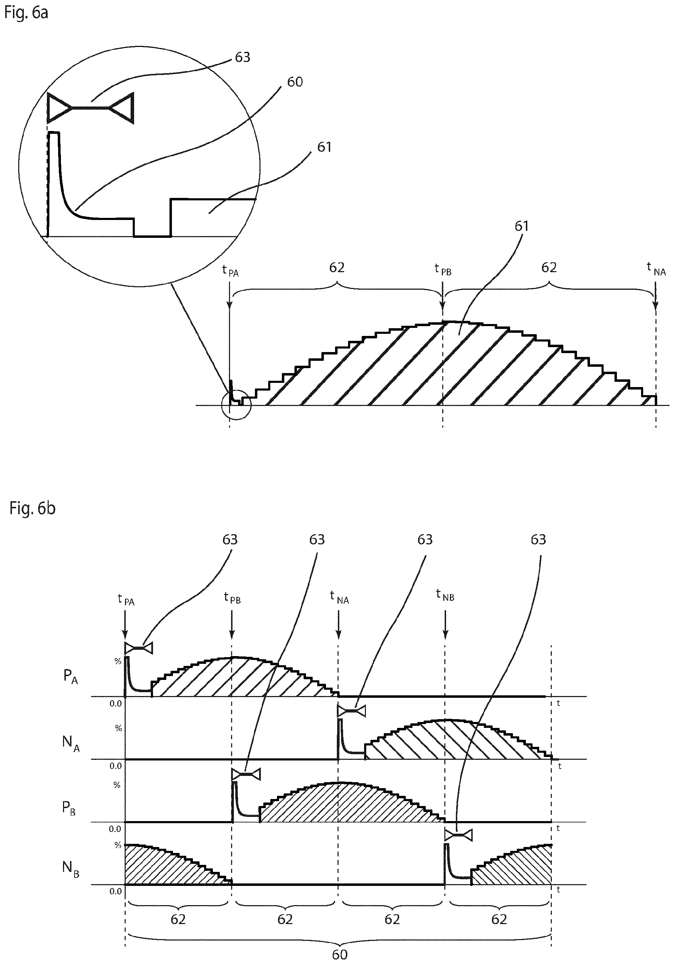

It is common practice in the design of brushless DC and/or stepper motor control to divide the driving time for a specific driving contact into a number of micro-steps, to vary the driving voltage with every micro-step and to spread a pattern of driving voltages over the entire commutation cycle. This means that the driving phases overlap and the driving voltages can be optimised for smoother and more energy efficient commutation. Fig. 6a shows an example of such a driving pattern, with a shape of half a sine wave spread over half of a commutation cycle, illustrated with 16 micro-steps per motor step and four motor steps per commutation cycle. In this example, the measuring window is positioned at the beginning of the first micro-step of the commutation pattern, and the course of Back EMF voltages sampled during the measuring window is shown enlarged. For commutation, the same driving pattern including the same measuring window is applied cyclically to all four driving contacts, as shown in Fig. 6b. In this example, all patterns consist of positive voltages. With every phase having a positive driving contact and a negative driving contact, the voltages on a particular phase could also be seen as a micro-stepped complete sine wave. It is, however, an important aspect of the present invention that positive driving contacts and negative driving contacts of all phases are treated separately as shown. The commutation pattern may have any other shape, such as rectangular or trapezoidal, and the measuring window may be positioned anywhere in the commutation pattern for any duration. To perform the measurement with the least possible consequence for commutation, however, it is advantageous to choose a time for the voltage measurement where no commutation is active, or, in the language of the example in Fig. 6b, a time where the driving voltage pattern is absent or has zero volt. This is the case whenever the polarity of a phase changes, in other words whenever the control circuitry switches the driving pattern from the positive driving contact of a phase to the negative driving contact of the same phase, or vice versa. In the preferred embodiment with two phases and four driving contacts in the commutation cycle, there will be two such switching events per phase and commutation cycle, which is the same as one switching event per driving pattern of each driving phase. In the example of a micro-stepped half sine wave, there is one micro-step with zero volt, which is either at the beginning or at the end of every pattern, depending on where the start of a cycle is defined to be. In Fig. 6a, the measuring window (63) for Back EMF voltage measurement starts with every driving voltage pattern (61), and every driving voltage pattern (61) starts with one micro-step of zero voltage, which means that the Back EMF measurement can take place without modifying the commutation. In Fig. 6a the duration of the measuring window (63) is shown to be shorter than one micro-step. This is not necessarily always the case, as will be explained further below. Fig. 7 shows a further detailed view of the voltage already enlarged in Fig. 6a, giving an example of a course of voltage measured on the driving contacts of phase A just before beginning to apply a driving voltage pattern, during a measuring window (63) of 0.2ms with no driving voltage active. The voltage at the driving contacts of phase A in this situation is a combination of voltage induced by switching off coil A (40) and voltage induced in the same coil by the movement of the rotor. As illustrated in Fig. 7, the voltage at or voltage difference between the driving contacts immediately jumps to a high value, due to switching off commutation at the opposite end of the coil (40), then quickly and at least partly exponentially falls down to converge towards a substantially horizontal part of the curve, oscillating around a substantially constant value: the convergence value (80, see Fig. 8). The convergence value is a basis for all subsequent analysis an. As an obvious alternative, instead of measuring the voltage at a driving contact, the electrical current at a driving contact could be measured and analysed.

In a preferred embodiment of the present invention, the convergence value is determined by sampling, during each measuring window (63), the course of the voltage at a driving contact (41) or the course of the voltage difference between two driving contacts (41) of the paused driving phase, and storing the measured values in a set of convergence data (71), starting at a convergence offset (70) from the beginning of the measuring window (63). Sampling is typically performed by triggering the ADC in the measuring circuitry at a constant sampling rate. In a preferred embodiment, the drug delivery device operates with a sampling rate of at least 1kHz, preferably at least 1MHz, and the convergence data consist of 1 to 1000, preferably 16 convergence data values obtained at a convergence offset of 0 to 1000, preferably 184 samples within the measuring window. The duration of the measuring window (63) required to let the initial peak pass and determine the convergence value depends on the inductor time constant Tau of the stator coils, on the driving voltage, on the rotation speed of the motor, on the implementation of the measurement circuitry, and on other factors. No minimum offset can hence be specified for the general method. Choosing a longer measurement window may increase, to a limit, the accuracy of calculating the convergence value, but the disadvantages of pausing the commutation will soon become prohibitive. In a practical realisation of the present invention, the course of the Back EMF voltage will be analysed for a particular motor, a particular control circuitry and a particular measurement circuitry, and the duration of the measurement window will be adjusted accordingly. As discussed above, an ideal Back EMF voltage measurement is designed with as little as possible impact on commutation. However, in many cases the duration of the measuring window required for Back EMF voltage analysis will exceed the duration of a micro-step, at least under certain operating conditions, for example at high speed or if the driving voltage pattern is applied with finely pitched micro-steps. To run the blockage detection, the measuring window will typically get priority over commutation at least on a regular selection of commutations, preferably for all applications of a driving voltage pattern. This is illustrated in Fig. 6b, where the measuring windows (63) are visibly longer than even several micro-steps.

To arrive at a single convergence value per measuring window, the voltage measurement circuitry will preferably sample the course of the Back EMF voltage at the driving contacts of a driving phase over at least part of the measuring window and evaluate a multitude of measured voltages. In a preferred embodiment of the blockage detection according to the present invention, a set of convergence data (71), a selection of sampled voltage values starting at a convergence offset (70) after start of the measuring window (63), is used to determine the convergence value (80) of that particular measuring window. In the example of Figure 7, the last 16 values sampled in a measuring window are selected as a set of convergence data and used to calculate the convergence value. This process is illustrated in Figure 8. The convergence value of the measuring window is preferably calculated as an average or arithmetic mean of all convergence values in the set of convergence data, although it could also be any other mathematical method to get a value representative of the measuring window, such as applying a digital filter, a statistical analysis or any kind of selection. Step 3 of the blockage detection, in all these cases, includes at least one convergence value per measuring window.

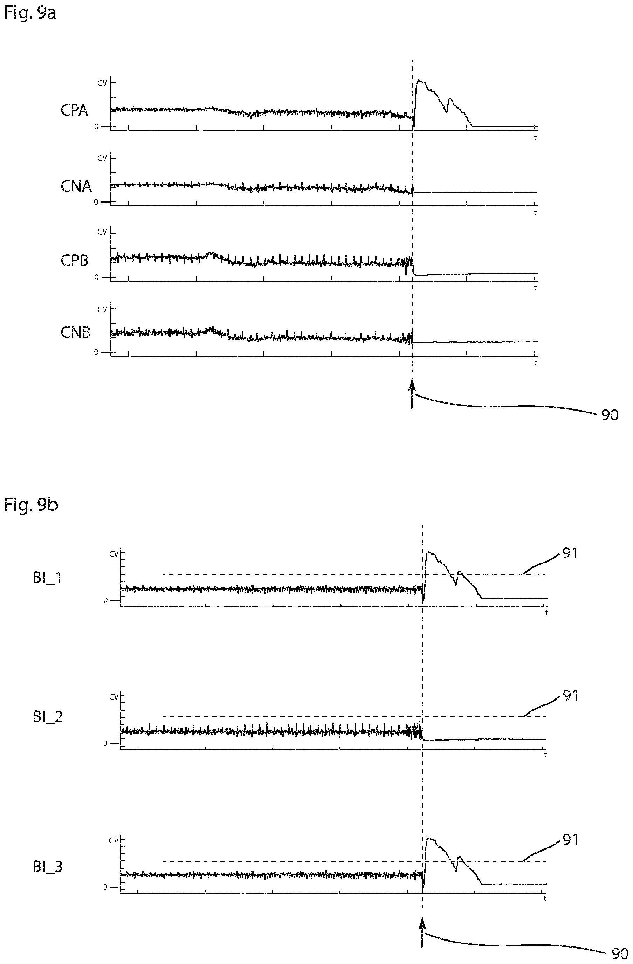

In step 4 of the blockage detection, the divergence of convergence values assigned to different driving contacts is quantified by calculating a blockage index BI. To specify a mathematical expression for a particular embodiment of the blockage detection, it may be helpful to assign each convergence value acquired in steps 1 to 3 to one of the driving contacts. As the Back EMF voltage at the contacts of a coil changes polarity when a driving voltage is switched off, and as the convergence values are preferably calculated from positive voltages, the most natural assignment is to assign the convergence values derived from voltage measurements on a positive driving contact to the negative driving contact of the same phase, and the convergence values derived from voltage measurements on a negative driving contact to the positive driving contact of the same phase. This is the logic preferably applied when implementing the present invention. Other assignment rules could be used, for example to the same driving contact as the measurement was taken from, provided that values from different driving contacts are consistently kept separate from each other and the assignment allows the analysis of the course of convergence values over a multitude of commutation cycles. The assignment of convergence values to a driving contact is preferably realised by storing convergence values in separate storage elements so that further processing steps can access the values selectively. The blockage detection according to the present invention preferably includes the use of multiple convergence values assigned to the same driving contact over a plurality of commutation cycles. Consequently, the assigning step will preferably include storing sequences of convergence values, preferably one sequence per driving contact. In the example of a preferred embodiment with two phases A and B, four driving contacts $P_A$, $N_A$, $P_B$, $N_B$ and one measurement window per application of a driving pattern, there are two sequences $CP_A$, $CP_B$, each with a sequence of convergence values assigned to a positive driving contact $P_A$, $P_B$, and two sequences $CN_A$ $CN_B$, each with a sequence of convergence values assigned to a negative driving contact $N_A$, $N_B$. Every driving contact is measured once per commutation cycle, hence said sequences typically contain one convergence value per commutation cycle. The term "sequence", in the context of convergence values, refers to the fact that one particular set of data is updated for a succession of commutation cycles. The number of convergence values stored in a storage element holding a sequence depends on the implementation of the method as described in the present invention. Figure 9a shows the sequences $CP_A$, $CN_A$, $CP_B$ and $CN_B$ in a graphical representation over a time of several minutes. The arrow (110) marks the time of blockage of the actuating assembly, in the example of the preferred embodiment the time of blockage of the plunger. It is clearly visible that the sequence $CP_A$ shows a significantly different course of convergence values after blockage than the sequence $CN_A$. The reason for this effect is that, in an actuating system with a minimum (non zero) elasticity in the actuating assembly, the rotor does not immediately get stuck and motionless in case of a blockage. The rotor would move forward until the maximum torque is reached, then fall back a small angle when the commutation recedes. This angular motion of the rotor causes a Back EMF signal. When in this situation the commutation is continued and a commutation pattern is applied to the opposite end of the same phase, the Back EMF signal will be different, typically close to zero, because the rotor will not be in the correct angular position to be advanced by a positive commutation. The rotor may even be reversed one step. Different scenarios of rotor movement at maximum torque in the presence of elasticity of the actuating assembly can be visualised using the schematic illustration of the magnetic structure of the motor in Figures 4a to c. They all lead to a divergence of Back EMF signals measured at opposite ends of the same phase, consequently to a divergence of convergence values assigned to both the positive and the negative driving contact of the same phase, and further consequently to a divergence of convergence values assigned to any selection of different driving contacts, provided said selection contains the positive and the negative driving contact of at least one phase.

As a main part of step 4, the blockage index is derived from the convergence values. The blockage index may be obtained explicitly by calculating a mathematical expression including or corresponding to a subtraction of values assigned to different driving contacts or groups of driving contacts, or implicitly by applying a statistical analysis or comparison of maximum and minimum values over all driving contacts. Fig. 9b shows the graphs of three basic examples of the blockage index BI in an embodiment with two phases A and B:

$$BI\_1(k) = CP_A(k) - CN_A(k)$$

or

$$BI\_2(k) = CP_B(k) - CN_B(k)$$

or

$$BI\_3(k) = \text{maximum}(CP_A(k), CN_A(k), CP_B(k), CN_B(k))$$
$$- \text{minimum}(CP_A(k), CN_A(k), CP_B(k), CN_B(k))$$

if k is a rumming number of communication cycles.

As the divergence of convergence values is especially surprising when observed between convergence values assigned to opposite ends of the same phase, the selection of driving contacts included in calculating the blockage index will typically include both ends of every phase selected, preferably both ends of all phases used to drive the motor. Calculating a blockage indication representing the divergence of positive and negative convergence values is not limited to mathematical expressions involving the difference operator, but could be any other means of expressing the difference between the sequences, for example assigning ranges of values, using statistical analysis or applying thresholds, just to name a few. Calculating a blockage indication may also use a combination of explicit and implicit differences, for example by calculating the difference between an actual convergence value and a statistical value like a mean convergence value. Just like the sequences of convergence values, the blockage indication may also be stored sequentially in a storage element of sufficient length to allow further processing, realising another sliding window of at least one, preferably at least ten commutation cycles.

As step 5 of blockage detection, the drug delivery device uses a blockage indication and a blockage criterion to decide if a blockage of the actuation assembly is present. Again, numerous ways exist to specify a criterion, and this criterion obviously depends on how the blockage indication is calculated. Using the example of a simple difference of convergence values on a single phase, exceeding a threshold would be an equally simple, preferred blockage criterion: Blockage = YES if BI_I(k) > ±threshold (91) with threshold in the range of, for example, 3 to 6, if using the convergence values as shown in Fig. 9b.

A more robust blockage detection uses multiple blockage indicators calculated at different times, for example by waiting with a blockage decision until threshold (91) has been exceeded multiple times, in a preferred embodiment at least three times in the last ten commutation cycles. Similar to the calculation of the blockage indication, the blockage criterion is not limited to a mathematical expression, but could be any other means of taking a decision based on the blockage indication calculated according the method of the present invention, for example by using ranges of values, using statistical analysis or applying variable thresholds, just to name a few.

Once the drug delivery device has reached the decision that a blockage of the actuating assembly is present, the device will forward this information to the patient or to another external component involved in controlling the drug delivery. This is step 6 of the blockage detection and will typically include an alarming action to attract external attention. In a simple embodiment, the alarming is done by changing the status of the drug delivery device, and by a control element reading the status and reacting on the change. Other alarming actions could be a visual or acoustic alarm on the drug delivery device itself, or to communicate the blockage to an external device for further control of the reaction, for example to display a warning on a remote control device or smartphone. Alternatively or additionally, the drug delivery device may react to the blockage with a mitigation action, such as stopping the commutation or reversing the commutation to unblock the actuating assembly, or with providing guidance to the user to effectuate further action.

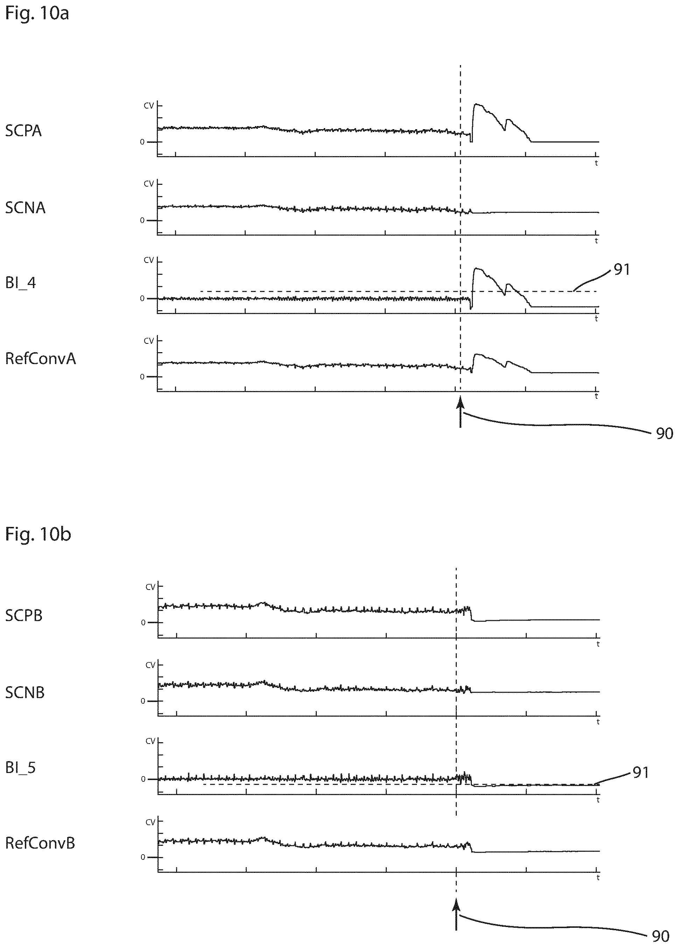

Starting from a drug delivery device with system for blockage detection as described with steps 1 to 6, a number of further improvements can be included. A first further aspect of the present invention relates to smoothing of the convergence values. Looking at Fig. 9a, it is apparent that convergence values, even if they are already a result of averaging of a multitude of convergence data values, may show ripples, small oscillations or similar variations when comparing convergence values of subsequent commutation cycles. As a basis for a detection of blockage it is advantageous to use convergence values with less short-term variation to enhance the contrast to a longer-term change. In a further preferred embodiment of the current invention, the convergence values are therefore smoothed over a time of more than one, preferably at least 16 commutation cycles, by applying a digital filter and/or calculating a sliding arithmetic mean, a sliding weighted mean, a sliding median or other mathematical method of smoothing a series of values. The resulting graph of smoothed convergence values looks smoother in short-term variation with a pronounced change when the actuating assembly is blocked. This is illustrated in Fig. 10a (phase A) and Fig. 10b (phase B), where $SCP_A$, $SCN_A$, $SCP_B$ and $SCN_B$ show smoothed variants of the sequences $SCP_A$, $SCN_A$, $SCP_B$ and $SCN_B$. The calculation of a blockage index may remain the same as with original convergence values. Still in Figure 10, further examples of blockage indices are shown as

$$BI\_4(k) = SCP_A(k) - SCN_A(k)$$

or

$$BI\_5(k) = SCP_B(k) - SCN_B(k)$$

with k still as a running number of commutation cycles.

A second further aspect of the present invention relates to normalisation of convergence values. As the blockage detection is based on differences, and differences can always be expressed as absolute values or else as relative deviations from a reference, a further preferred embodiment of the present invention includes calculating a sliding reference value RefConv, and expressing all convergence values as relative deviations from said reference RefConv. In a preferred embodiment there is one reference per phase. A more global reference could include RevConv being calculated globally from all convergence values measured during a series of commutation cycles. A simple example of generating a sliding reference is to calculate, for each phase and commutation cycle, the arithmetic mean of the convergence value assigned to the positive driving contact and the convergence value assigned to the negative driving contact. Figures 10a and 10b contain graphs for $RefConv_A$ and $RefConv_B$ which are the sequences of the sliding average of $SCP_A$ and $SCN_A$, and of $SCP_B$ and $SCN_B$ respectively. Normalisation is typically performed by dividing each convergence value by the reference value for the same commutation cycle, as for example in the formula

$$N\_SCP_A(k) = SCP_A(k) / RefConv_A$$

with a running number of commutation cycles k, and $N\_SCP_A(k)$ being a normalised form of the smoothed convergence value $SCP_A$. Normalised convergence values my further be scaled, for example to represent a percentage, as in

$$N\_SCP_A(k) = SCP_A(k) \, / \, RefConv_A * 100$$

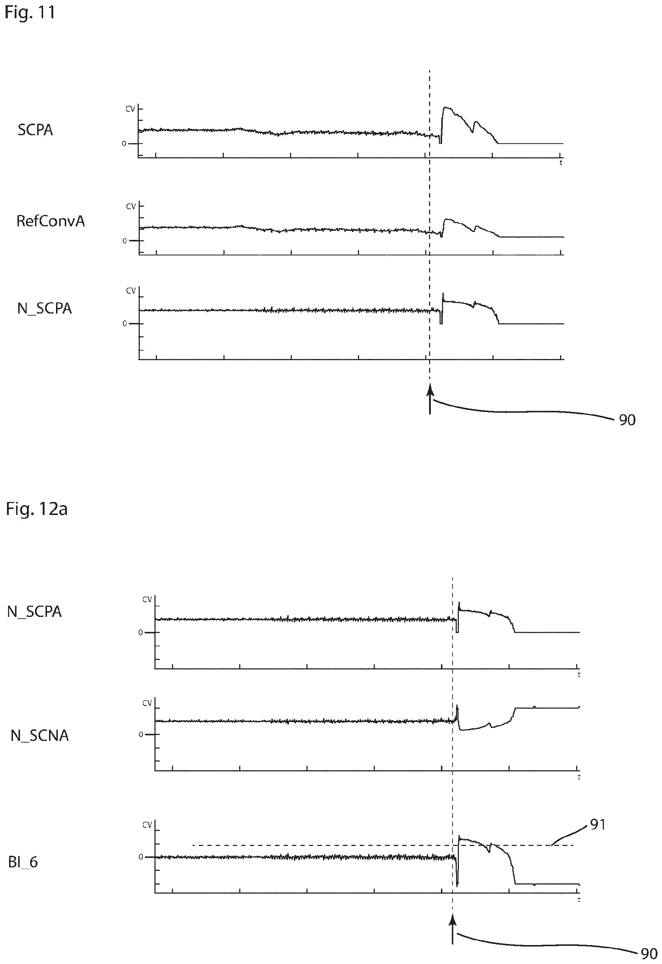

Figure 11 illustrates the example of normalisation of the sequence $SCP_A$ as just described.

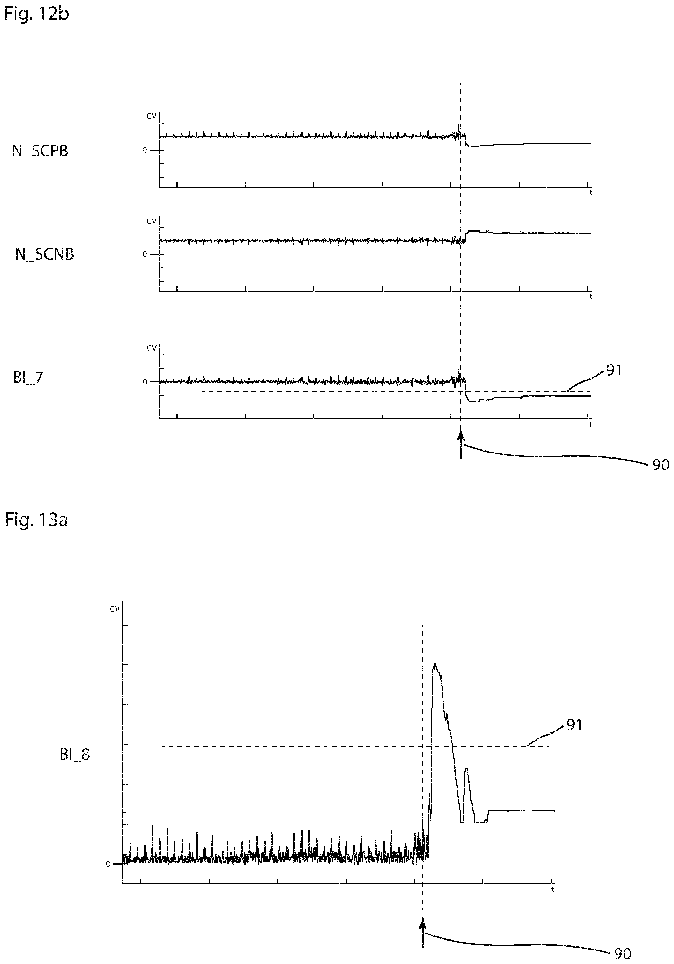

To avoid division by zero when calculating a normalised convergence value, a small non-zero value may be added to the reference RevConv. If an embodiment of the blockage detection includes smoothing of convergence values, the normalisation may take place either before or after performing the smoothing, the latter being preferred for easier implementation. The steps of smoothing a series of convergence values, calculating a reference and normalising the convergence values could also be performed in one step for optimum efficiency in implementation. Just like unprocessed or smoothed convergence values, normalised convergence values may be used to calculate a blockage indication value based on differences of values assigned to positive driving contacts and values assigned to negative driving contacts. Figure 12b (bottom) shows a graph of the following example:

$$BI\_6(k) = N\_SCP_A(k) - N\_SCN_A(k)$$

$$BI\_7(k) = N\_SCP_B(k) - N\_SCN_B(k)$$

with k as a running number of commutation cycles

As seen when discussing Fig. 9, the difference between convergence values assigned to positive driving contacts and convergence values assigned to negative driving contacts typically appears in one driving phase only. In a system with more than one driving phase, the angular position the rotor in a status of blockage determines which driving phase is showing the difference between convergence values assigned to positive and negative driving contacts. Hence, a third further aspect of the present invention relates to calculating the total blockage indication value based on a combination of blockage indices individually calculated for different phases. This extension ensures a reliable blockage detection in a system with more than one driving phase. A simple example of such a combination is

$$BI\_8(k, total) = maximum \, ( \, BI\_4(k), BI\_5(k) \, )$$

or

$$BI\_8(k) = maximum \, ( \, SCP_A(k) - SCN_A(k), SCP_B(k) - SCN_B(k) \, )$$

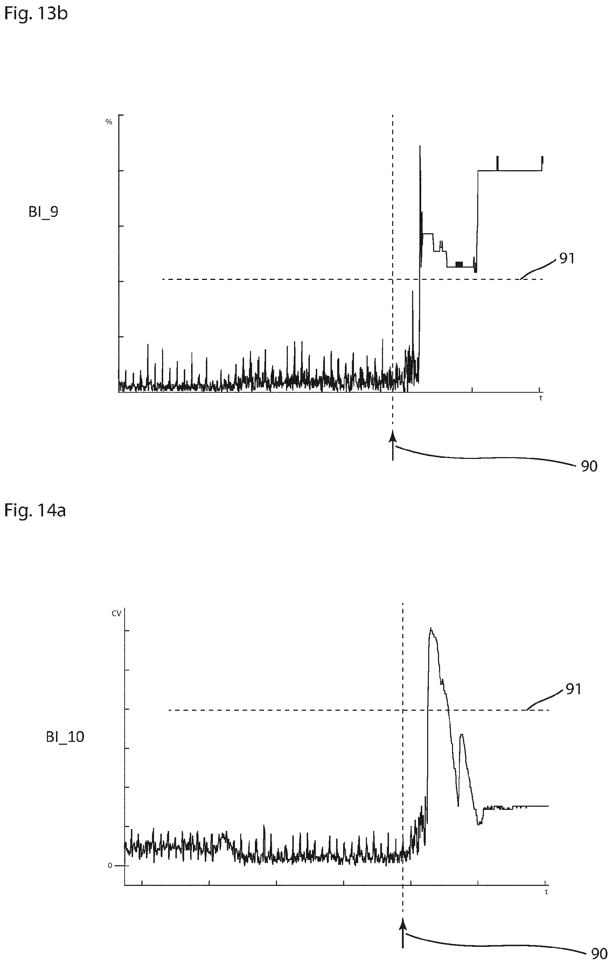

again with k as a running number of commutation cycles, see Figure 13a. The same combination of phases may of course be calculated from normalised convergence values, as shown in Figure 13b and expressed in the formula

$$BI\_9(k) = maximum \, (N\_SCP_A(k) - N\_SCN_A(k), N\_SCP_B(k) - N\_SCN_B(k))$$

A fourth further aspect of the present invention relates to calculating the blockage indication value based on a combination of convergence values from a multitude of phases rather than from precalculated blockage indices from individual phases. The divergence of convergence values may be differences between values assigned to both ends of the same phase, but the same divergences may be visible, and quantifiable, by analysing the convergence values as assigned to any suitable combination of the driving contacts. One example of such a blockage indication value has already been mentioned as BI_1(k), looking for the maximum variation of values over all convergence values as assigned to any driving contacts. This aspect of the present invention includes all sorts of statistical analysis or method for comparison in a set of values. Further simple examples of blockage indication values based on unsorted combinations of driving contacts are variations of BI_1 using sequences of smoothed convergence values SCP, SCN or sequences of normalised convergence values N_SCP, N_SCN:

$$BI\_10(k) = \text{maximum } (SCP_A (k), SCN_A (k), SCP_B (k), SCN_B (k))$$
$$- \text{minimum } (SCP_A (k), SCN_A (k), SCP_B (k), SCN_B (k))$$

and

$$BI\_11(k) = \text{maximum } (N\_SCP_A (k), N\_SCN_A (k), N\_SCP_B (k), N\_SCN_B (k))$$
$$- \text{minimum } (N\_SCP_A (k), N\_SCN_A (k), N\_SCP_B (k), N\_SCN_B (k)).$$

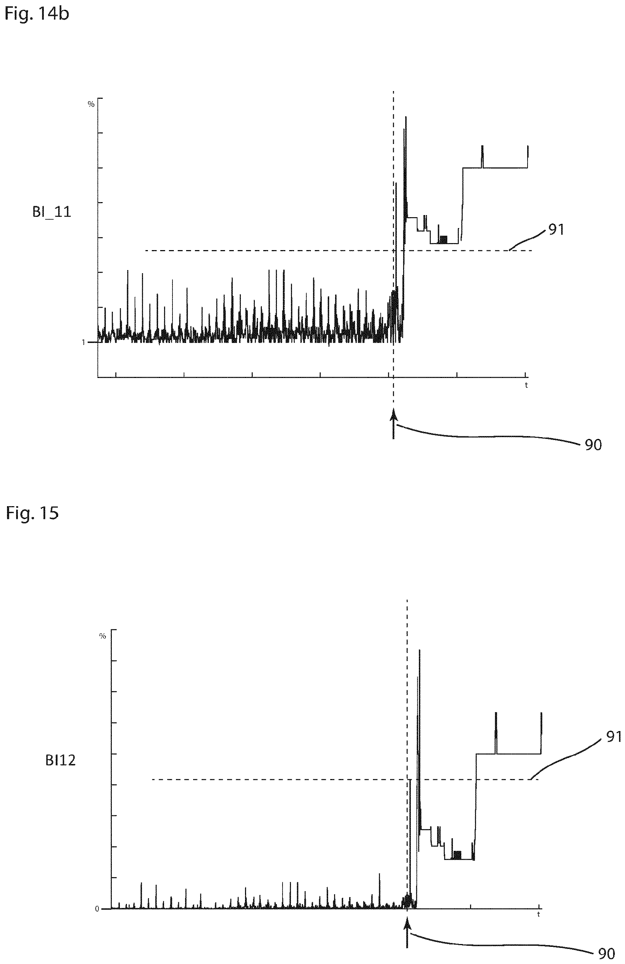

as shown in Figure 14a and Figure 14b.

Changing the optic from looking at absolute differences to looking at relative deviations or errors, a fifth further aspect of the present invention emerges, relating to calculating the blockage indication value as an error or deviation from a normalisation reference as described before. Just like selecting convergence values from multiple phases and combining them to a global blocking indication value, the same concept may be applied to normalisation. The reference used for normalisation may be calculated from multiple phases, stored from earlier commutation cycles, programmed as a fixed constant or obtained in another way to serve the purpose of indicating a deviation of convergence values assigned to positive driving contacts from convergence values assigned to a negative driving contact. When indicating a comparison with a reference, the blockage indication value may be interpreted as an indicator of an error of convergence values. Again, to give a simple example, a blockage indication value may be calculated as the maximum of normalised convergence values for all driving contacts:

$$BI\_12(k) = \text{maximum } (N\_SCP_A (k), N\_SCN_A (k), N\_SCP_B (k), N\_SCN_B (k))$$

However, in BI_12, a large negative error value would be ignored. A preferred further embodiment of a blockage indication hence is to get rid of the leading sign of the convergence value and use the concept of a mean square error. Figure 15 shows a graph of a blockage indication value with the formula

$$BI\_13(k) = \text{maximum } ((N\_SCP_A (k))^2, (N\_SCN_A (k) )^2, (N\_SCP_B (k) )^2, (N\_SCN_B (k) )^2).$$

Obviously, using the absolute value of error values would lead to a similar result.

While the description of possible embodiments of the present invention in this document has focused on keeping the calculation of the blockage indication values as simple as possible, all sorts of more complicated embodiments may be used with the same effect. The blockage indication value may, for example, use processed or unprocessed convergence values from different commutation cycles rather just one, patterns of values or characteristics of curves may be analysed, longer term behaviour analysis could be included or variation over time like using a non-constant sensitivity for blockage detection. Common to all these variations is that the blockage detection is based on an analysis of differences in Back EMF voltages observed on positive driving contacts and Back EMF voltages observed on negative driving contacts. It is evident that "differences" may not explicitly be expressed as a subtraction in a formula, but could implicitly be included in another mathematical concept like average, mean, correlation, drop, ratio, variance, just to name a few.

While most preferred embodiments have been explained using the example of a wearable patch injector as shown in figure 1, it is evident that the current invention may also be used in all sorts of pumps for the infusion of drugs like insulin, in handheld electronic injectors, or in any other kind of medical device, wherever a stepper or brushless DC motor is used to effectuate fluid delivery or actuate a mechanical movement which is relevant for a therapy and where hence a reliable blocking detection is advantageous.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

LIST OF REFERENCE NUMERALS

| | |
|---|---|
| 1 | Patch pump / fluid delivery system |
| 10 | Housing |
| 11 | Command button |
| 12 | Reservoir window |
| 13 | Adhesive patch assembly |
| 20 | Electronic control unit |
| 21 | Reservoir |
| 22 | Plunger |
| 30 | Plunger moving assembly / Actuating assembly |
| 30a | Gear box assembly |
| 30b | Threaded rod |
| 30c | Segmented rod |
| 30d | Plunger moving head |
| 31 | Stepper or BLDC motor |
| 31a | Driving contact |
| 32 | Fluid transport assembly |
| 32a | Input |
| 32b | Output |
| 40 | Coil |
| 40a | Positive end |
| 40b | Negative end |
| 41 | Positive driving contact |
| 42 | Negative driving contact |
| 43 | Positive magnetic flux conductor |
| 44 | Negative magnetic flux conductor |
| 45 | Rotor |
| 50 | Control circuitry |
| 51 | MOS-FET switch |
| 55 | Voltage measurement circuitry |
| 60 | Back EMF signal |
| 61 | Driving voltage pattern |
| 62 | Motor full step |
| 63 | Measuring window |
| 70 | Convergence offset |
| 71 | Convergence data |
| 80 | Convergence value |
| 90 | Time of blockage |
| 91 | Threshold |

**Claims**

**1.** A method to detect and report blockage of an actuating assembly (30) in a mobile or wearable drug delivery device, whereby
the drug delivery device comprises an electric stepper motor or brushless DC motor (31) operatively connected to the actuating assembly (30) and comprising at least one driving phase, preferably two driving phases, each driving phase having a coil (40), a positive driving contact (41), and a negative driving contact (42) to drive the motor (31);
the drug delivery device comprises a voltage measurement circuitry (55) to measure the voltage at or between the driving contacts (41, 42);
the drug delivery device comprises an electronic control unit (20) with a control circuitry (50), an electrical power source, a microprocessor, storage and software configured to control the commutation of the motor (31), to supervise the actuation of the drug delivery device and to initiate an alarming and/or mitigation action if a blockage of the actuating assembly (30) has been detected;
the method comprises the steps of

commutating the motor (31) by applying a pattern of positive driving voltages (61) to the driving contacts (41), preferably repeating a commutation cycle with a succession of all positive driving contacts (41) and all negative driving contacts (42);

pausing the driving of at least one driving phase periodically, preferably twice per commutation cycle, further preferably pausing between driving the positive and driving the negative driving contact of the same driving phase, to open a recurring measuring window (63) during which the driving contacts (41, 42) of the paused driving phase are controlled by the voltage measuring circuitry (55) to measure a Back EMF voltage at or between said driving contacts (41, 42);

measuring, preferably during each measuring window (63), the voltage at or between driving contacts (41, 42) of a driving phase and establishing, preferably once for each measuring window (63), a convergence value (80) representing the Back EMF voltage during said measuring window (63);

calculating, preferably once for every commutation cycle k, a blockage indication value BI based on the divergence of convergence values measured at different driving contacts (41, 42);

deciding that a blockage of the actuation assembly is present if the blockage indication value BI meets a predefined criterion, preferably if the blockage indication value BI exceeds a blockage threshold ±MaxConvDiff for a specified minimum number of commutation cycles k within a specified minimum blockage duration; and

initiating an alarming and/or mitigation action if a blockage has been detected.

2. A method according to claim 1, whereby the measuring step includes

sampling, during each measuring window (63), the course of the voltage at a driving contact (41, 42) or the course of the voltage difference between two driving contacts (41, 42) of the paused driving phase, and storing the measured values in a set of convergence data (71), starting at a convergence offset (70) from the beginning of the measuring window (63); and

calculating from each set of convergence data (71) one convergence value (80), for example by applying a first digital filter and/or calculating a first arithmetic mean, a first weighted mean or a first median of the values in the set of convergence data;

3. A method according to claim 1 or 2, whereby the calculating step includes, for the purpose of formulating the calculation of the blockage index, an assigning step which includes

assigning each convergence value (80) to a positive or to a negative driving contact (41, 42), preferably to the positive or to the negative driving contact of the driving phase paused to perform the voltage measurement, further preferably assigning the convergence value to the driving contact to which the driving pattern has last been applied before starting the measurement window the convergence value is representing;

storing the convergence values in sequences per driving contact (41, 42), preferably with driving phases A and B the sequences $CP_A$, $CP_B$ for convergence values at the positive driving contacts $P_A$, $P_B$ (41) and the sequences $CN_A$, $CN_B$ for convergence values at the negative driving contacts $N_A$, $N_B$ (42).

4. A method according to any of the preceding claims, whereby the calculating step includes calculating the blockage indication value BI for every commutation cycle k based on the difference between convergence values CP assigned to a positive driving contact (41) and convergence values CN assigned to a negative driving contact (42), as for example in the formula

$$BI(k) = CP_A (k) - CN_A (k) \text{ using values from phase A}$$

or

$$BI(k) = CP_B (k) - CN_B (k) \text{ using values from phase B.}$$

5. A method according to any of the preceding claims, whereby the calculating step includes

calculating, for each sequence of convergence values a sequence of smoothed convergence values, preferably $SCP_A$, $SCP_B$, $SCN_A$ and $SCN_B$ wherein the course of convergence values has been smoothed, for example by applying a second digital filter and/or calculating a sliding second arithmetic mean, a sliding second weighted mean or a sliding second median of a predefined number of values, preferably of 16 values in each sequence CP$_A$, CP$_B$, CN$_A$ and CN$_B$;
calculating the blockage indication value BI for every commutation cycle k based on the difference between smoothed convergence values at a positive driving contact (41), preferably a member of SCP$_A$ or SCP$_B$, and a smoothed convergence value at a negative driving contact (42), preferably a member of SCN$_A$ or SCN$_B$, as for example in the formula

$$BI(k) = SCP_A(k) - SCN_A(k)$$

or

$$BI(k) = SCP_B(k) - SCN_B(k).$$

6. A method according to any of the preceding claims, further comprising the step of normalising the convergence values or smoothed convergence values before using them to calculate the blockage indication value BI; whereby preferably
normalisation is performed by mathematically converting all smoothed convergence values to a representation of a relative deviation from a sliding reference Ref$_A$, Ref$_B$ calculated for each driving phase;
the reference Ref$_A$, Ref$_B$ is calculated as a sliding average, weighted mean, arithmetic mean or median of the smoothed positive convergence value SCP$_A$, SCP$_B$ and the smoothed negative convergence value SCN$_A$ , SCN$_B$ of each commutation cycle k;

each smoothed conversion value in SCP$_A$, SCN$_A$, SCP$_B$ and SCN$_B$ is normalised by calculating the percentual deviation from Ref$_A$ or Ref$_B$ as for example in Norm_SCP$_A$(k) $= (SCP_A(k) / Ref_A(k)) * 100;$

7. A method according to any of the preceding claims, whereby the blockage indication value is based on a combination of blockage indication values calculated for a plurality of individual driving phases, as for example in the following formula:

$$BI = max\ (SCP_A - SCN_{A;}\ SCP_B - SCN_B) > MaxConvDiff\ when\ using\ the\ smoothed\ convergence\ values,$$

or

$$BI = max\ (Norm\_SCP_A - Norm\_SCN_{A;}\ Norm\_SCP_B - Norm\_SCN_B) > MaxConvError\ when\ using\ the\ normalised\ smoothed\ convergence\ values.$$

8. A method according to any of the preceding claims, whereby the calculation of the blockage indication value is based on a maximum difference of convergence values in a plurality of driving phases, as for example in the following formula:

$$BI = max\ (SCP_A;\ SCN_A;\ SCP_B;\ SCN_B) - min\ (SCP_A;\ SCN_A;\ SCP_B;\ SCN_B).$$

9. A method according to any preceding claim, whereby the calculation of the blockage indication value is based on a maximum deviation from a normalisation reference, preferably deviation from Ref$_A$, Refs, as for example in the following formula:

$$BI = max\ (Norm\_SCP_A;\ Norm\_SCN_A;\ Norm\_SCP_B;\ Norm\_SCN_B)$$

or

$$BI = \max \left( (\mathrm{Norm\_SCP_A})^2; (\mathrm{Norm\_SCN_A})^2; (\mathrm{Norm\_SCP_B}) \right)^2; (\mathrm{Norm\_SCN_B})^2 ).$$

10. A method according to any of the preceding claims, whereby the voltage measurement operates with a sampling rate of at least 1kHz, preferably at least 1MHz, and the convergence data consists of 1 to 1000, preferably 16 convergence data values obtained at a convergence offset of 0 to 1000, preferably 184 samples within the measuring window.

11. A system to detect and report blockage of an actuating assembly (30) in a mobile or wearable drug delivery device, whereby
the drug delivery device comprises an electric stepper motor or brushless DC motor (31) operatively connected to the actuating assembly (30) and comprising a driving phase, preferably two driving phases, each driving phase having a coil (40), a positive driving contact (41), and a negative driving contact (42) to drive the motor (31);
the system comprises a voltage measurement circuitry (55) to measure the voltage at or between the driving contacts (41);
the system comprises an electronic control unit (20) with a control circuitry (50), an electrical power source, a microprocessor, storage and software configured to control the commutation of the motor (31), to supervise the actuation of the drug delivery device and to initiate an alarming action if a blockage of the actuating assembly (30) has been detected;
***characterised by*** the blockage detection comprising the steps of
commutating the motor (31) by applying a pattern of positive driving voltages (61) to the driving contacts (41), preferably repeating a commutation cycle with a succession of all positive driving contacts (41) and all negative driving contacts (42);
pausing the driving of at least one driving phase periodically, preferably twice per commutation cycle, further preferably pausing between driving the positive and driving the negative driving contact of the same driving phase, to open a recurring measuring window (63) during which the driving contacts (41) of the paused driving phase are controlled by the voltage measuring circuitry (55) to measure a Back EMF voltage at or between said driving contacts (41, 42);
measuring, preferably during each measuring window (63), the voltage at or between driving contacts (41, 42) of a driving phase and establishing, preferably once for each measuring window, a convergence value (80) representing the Back EMF voltage during said measuring window (63);
assigning the convergence value (80) to a positive or to a negative driving contact (41, 42), preferably to the positive or to the negative driving contact of the driving phase paused to perform the voltage measurement, further preferably assigning the convergence value to the driving contact (41, 42) to which the driving pattern has last been applied before starting the measurement window the convergence value is representing;
calculating, preferably once for every commutation cycle k, a blockage indication value BI based on the divergence of convergence values assigned to different driving contacts;
deciding that a blockage of the actuation assembly is present if the blockage indication value BI meets a predefined criterion, preferably if the blockage indication value BI exceeds a blockage threshold MaxConvDiff for a specified minimum number of commutation cycles k within a specified minimum blockage duration.

12. A system according to claim 11, whereby the voltage measurement operates with a sampling rate of at least 1kHz, preferably at least 1MHz, and the convergence data consists of 1 to 1000, preferably 16 convergence data values obtained at a convergence offset of 0 to 1000, preferably 184 samples within the measuring window.

13. A system according to any of the claims 11 to 12, whereby the drug delivery device is a drug delivery device with a reservoir (21) for containing a medical fluid, a plunger (22) movably disposed in the reservoir (21) and a fluid transport assembly (32) to connect the reservoir to a patient, and whereby the actuating assembly (30) is a plunger moving assembly to move the plunger (22) in the reservoir (21) and effectuate fluid delivery to the patient.

Fig. 1

Fig. 2

Fig. 3

Fig. 4a

Fig. 4b

Fig. 4c

Fig. 5a

Fig. 5b

Fig. 6a

Fig. 6b

Fig. 7

Fig. 8

Fig. 9a

Fig. 9b

Fig. 10a

SCPA

SCNA

BI_4 ........................................ 91

RefConvA

↑ ........................................ 90

Fig. 10b

SCPB

SCNB

BI_5 ........................................ 91

RefConvB

↑ ........................................ 90

Fig. 11

SCPA

RefConvA

N_SCPA

90

Fig. 12a

N_SCPA

N_SCNA

BI_6

91

90

Fig. 12b

N_SCPB

N_SCNB

BI_7

91

90

Fig. 13a

BI_8

91

90

Fig. 13b

BI_9

Fig. 14a

BI_10

Fig. 14b

Fig. 15

Europäisches
Patentamt

European
Patent Office

Office européen
des brevets

# EUROPEAN SEARCH REPORT

Application Number

EP 20 20 5379

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/164142 A1 (POETSCHKE MARKUS [DE]) 28 May 2020 (2020-05-28) | 1-3,6-13 | INV.<br>H02P29/024<br>H02P6/08<br>H02P6/182<br>H02P8/38<br>A61M5/142 |
| A | * paragraph [0003] - paragraph [0008]; figures 1,2 *<br>* paragraph [0085] - paragraph [0093] *<br>* paragraph [0106] *<br>* paragraph [0033] * | 4,5 | |
| Y | WO 2011/073742 A2 (GALIC GORDAN [HR]; FIOLIC GORAN [HR]) 23 June 2011 (2011-06-23) | 1-3,6, 10-13 | |
| A | * paragraph [0002] - paragraph [0004]; figures 1-12 *<br>* paragraph [0041] - paragraph [0067] * | 4,5 | |
| Y | CN 110 187 274 A (VIVO COMM TECHNOLOGY CO LTD) 30 August 2019 (2019-08-30) | 1,3,6-9, 11,13 | |
| A | * paragraph [0045] - paragraph [0075]; figures 1-4 * | 4,5 | |
| A | EP 3 633 845 A1 (HANGZHOU SANHUA RES INST CO LTD [CN]) 8 April 2020 (2020-04-08)<br>* paragraph [0030] - paragraph [0077]; figures 3-9 * | 1-13 | TECHNICAL FIELDS SEARCHED (IPC)<br><br>H02P<br>A61M<br>H02H |
| A | EP 1 760 875 A1 (PROTRONIC N V [BE]) 7 March 2007 (2007-03-07)<br>* paragraph [0017] - paragraph [0027]; figures 1-10 *<br>* paragraph [0033] - paragraph [0035] * | 1-13 | |

The present search report has been drawn up for all claims

| Place of search | Date of completion of the search | Examiner |
|---|---|---|
| Munich | 16 April 2021 | Gospodinova, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 20 20 5379

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2021

| Patent document cited in search report | | Publication date | Patent family member(s) | | Publication date |
|---|---|---|---|---|---|
| US 2020164142 | A1 | 28-05-2020 | AR | 112805 A1 | 18-12-2019 |
| | | | AU | 2018311202 A1 | 30-01-2020 |
| | | | BR | 112020001270 A2 | 21-07-2020 |
| | | | EP | 3662574 A1 | 10-06-2020 |
| | | | JP | 2020529245 A | 08-10-2020 |
| | | | US | 2020164142 A1 | 28-05-2020 |
| | | | WO | 2019025591 A1 | 07-02-2019 |
| WO 2011073742 | A2 | 23-06-2011 | US | 2011181229 A1 | 28-07-2011 |
| | | | WO | 2011073742 A2 | 23-06-2011 |
| CN 110187274 | A | 30-08-2019 | CN | 110187274 A | 30-08-2019 |
| | | | WO | 2020238582 A1 | 03-12-2020 |
| EP 3633845 | A1 | 08-04-2020 | CN | 108931085 A | 04-12-2018 |
| | | | EP | 3633845 A1 | 08-04-2020 |
| | | | JP | 2020518224 A | 18-06-2020 |
| | | | KR | 20190127901 A | 13-11-2019 |
| | | | US | 2020161996 A1 | 21-05-2020 |
| | | | WO | 2018214801 A1 | 29-11-2018 |
| EP 1760875 | A1 | 07-03-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0341364 B1
- US 20030117100 A1
- US 9509243 B2
- WO 2017017557 A1